# EUROPEAN PATENT APPLICATION

(11) **EP 1 094 110 A1**
(43) Date of publication of application: **25.04.2001**
(21) Application number: 99203467.8
(22) Date of filing: 21.10.1999
(51) Int. Cl.: C12N 15/34, C07K 14/01, C07K 16/08, A61K 39/12

(54) **Apoptosis inducing proteinaceous substance**

(71) Applicant: Leadd B.V., 2333 AL Leiden (NL)
(72) Inventor: Noteborn, Mathieu Hubertus Maria, 2352 EH Leiderdorp (NL)
(74) Representative: Ottevangers, Sietse Ulbe

(57) **Abstract**

The invention relates to the field of apoptosis. The invention provides novel therapeutic possibilities, for example novel combinatorial therapies or novel therapeutic compounds that can work alone, sequentially to, or jointly with apoptin, especially in those cases wherein p53 is (partly) non-functional and/or Bcl-2 is overexpressed.

## Description

The invention relates to the field of apoptosis.
Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).
The apoptotic process can be initiated by a variety of regulatory stimuli (Wyllie, 1995, White 1996, Levine, 1997). Changes in the cell survival rate play an important role in human pathogenesis, e.g. in cancer development and auto-immune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).
Many tumors, however, acquire a mutation in p53 during their development, often correlating with poor response to cancer therapy. Transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).
For such cancers (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by anti-apoptotic activities, such as Bcl-2 or Bcr-abl-like ones. These factors might be part of a distinct apoptosis pathway or being (far) downstream to the apoptosis inhibiting compounds.
Apoptin is a small protein derived from chicken anemia virus (CAV; Noteborn and De Boer, 1996, Noteborn et al., 1991, Noteborn et al., 1994; 1998a), which can induce apoptosis in human malignant and transformed cell lines, but not in untransformed human cell cultures. In vitro, apoptin fails to induce programmed cell death in normal lymphoid, dermal, epidermal, endothelial and smooth-muscle cells. However, when normal cells are transformed they become susceptible to apoptosis by apoptin. Long-term expression of apoptin in normal human fibroblasts revealed that apoptin has no toxic or transforming activity in these cells (Danen-van Ooschot, 1997 and Noteborn, 1996).
In normal cells, apoptin was found predominantly in the cytoplasm, whereas in transformed or malignant cells i.e. characterized by hyperplasia, metaplasia or dysplasia, it was located in the nucleus, suggesting that the localization of apoptin is related to its activity (Danen-van Oorschot et al. 1997).
Apoptin-induced apoptosis occurs in the absence of functional p53 (Zhuang et al., 1995a), and cannot be blocked by Bcl-2, Bcr-abl (Zhuang et al., 1995), the Bcl-2-associating protein BAG-1 and not by the caspase-inhibitor cowpox protein CrmA (Danen-Van Oorschot, 1997a, Noteborn, 1996). Therefore, apoptin is a particularly useful therapeutic compound for the destruction of tumor cells, or other hyperplasia, metaplasia or dysplasia which have become resistant to (chemo)-therapeutic induction of apoptosis, due to for example the lack of functional p53 and (over)-expression of Bcl-2 and other apoptosis-inhibiting agents (Noteborn and Pietersen, 1998).
It appears, that even pre-malignant, minimally transformed cells, are sensitive to the death-inducing effect of apoptin. In addition, Noteborn and Zhang (1998) have shown that apoptin-induced apoptosis can be used as diagnosis of cancer-prone cells and treatment of cancer-prone cells.
The fact that apoptin does not induce apoptosis in normal human cells, at least not in vitro, shows that a toxic effect of apoptin treatment in vivo will be very low. Noteborn and Pietersen (1998) and Pietersen et al. (1999) have provided evidence that adenovirus expressed apoptin does not have an acute toxic effect in vivo. In addition, in nude mice it was shown that apoptin has a strong anti-tumor activity.
However, to further enlarge the array of therapeutic anticancer or anti-auto-immune-disease compounds available in the art, additional therapeutic compounds are desired that are designed to work alone, sequentially to, or jointly with apoptin or other apoptosis inducing substances, especially in those cases wherein the tumor suppressor p53 is (partly) non-functional and or anti-apoptosis proteins such as Bcl-2 are over-expressed.
The invention provides novel therapeutic possibilities, for example novel combinatorial therapies or novel therapeutic compounds that can work alone, sequentially or jointly with apoptin, especially in those cases wherein p53 is (partly) non-functional and/or Bcl-2 is over-expressed.
In a first embodiment, the invention provides an apoptosis inducing proteinaceous substance, capable of providing apoptosis, alone or in combination with other apoptosis-inducing substances, such as apoptin, said proteinaceaous substance encoded by an isolated or recombinant nucleic acid or functional equivalent or fragment thereof derivable from a mammalian gyrovirus-like virus nucleic acid.

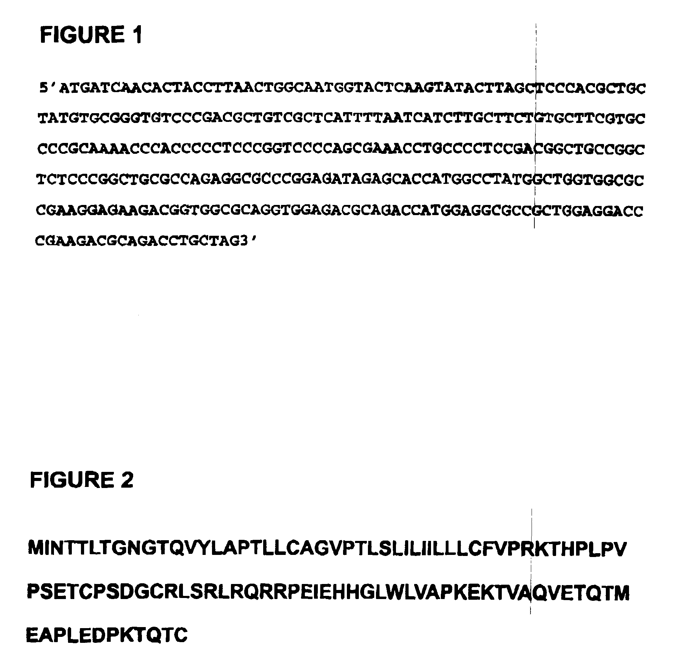

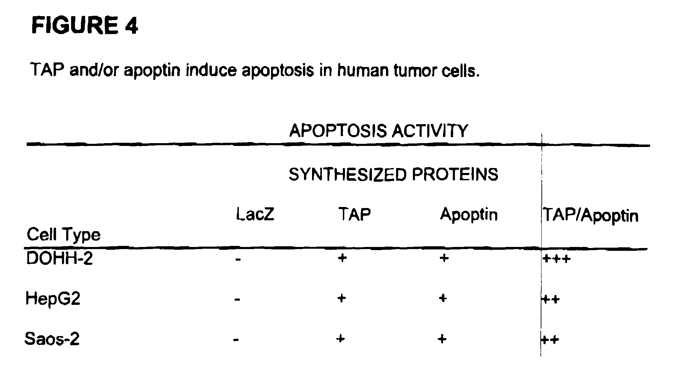

Mammalian gyrovirus-like viruses, which are circovirus-like single-stranded circular DNA viruses distinct from for example porcine circovirus or relatives thereof, have been found which surprisingly can be used to provide a substance providing apoptosis, thereby providing functional equivalent proteinaceous substances or fragments thereof having similar features as CAV-derived apoptin or fragments thereof. In particular, the invention provides a proteinaceous substance according to the invention wherein said mammalian gyrovirus-like virus comprises a single-stranded circular DNA virus, such as for example a TTV-like virus, such as seen with the recently discovered novel human single-stranded circular DNA virus TTV (Charlton et al., 1998; Miyata et al., 1999). In a preferred embodiment, the invention provides a proteinaceous substance or functional equivalent or fragment thereof, said substance encoded by an isolated or recombinant nucleic acid which is at least 60% homologous preferably at least 70%, more preferably at least 80%, even more preferably at least 90%, most preferably at least 95% to a TTV-like virus nucleic acid as shown in figure 1.
Surprisingly, such a proteinaceous substance according to the invention is capable of providing apoptosis alone or in combination with another apoptosis-inducing substance, as for example shown in figure 4, in particular wherein said apoptosis-inducing substance comprises apoptin or a functional fragment thereof. In a particular advantageous embodiment of the invention, siad proteinaceous substance according is capable of providing apoptosis independent of p53. Preferably, a proteinaceous substance according to the invention, or functional equivalent or fragment thereof, comprises a nuclear location signal and/or a nuclear export signal as for example shown in fig 2.
Proteinaceous substance, herein also called TAP protein, is defined as a substance comprising a peptide, optionally having been modified by for example glycosylation, myristilation, phosphorylation, the addition of lipids, by homologous or heterologous di- or multimerisation, or any other (posttranslational) modifications known in the art. TTV-derived protein or proteinaceous substance herein is defined as a protein for example encoded by strains of the recently discovered novel human single-stranded circular DNA virus TTV (Charlton et al., 1998; Miyata et al., 1999). Thus far, TTV and TTV-like viruses have not been correlated to any known disease. However, TTV contains also a single-stranded circular DNA which relates it to CAV, from which apoptin is derived. TTV has a similar genomic organization like CAV, however, none of the predicted open reading frames of TTV were proven to be transcribed and translated in TTV-infected cells (Miyata et al., 1999). In particular, the herein described TTV open reading frame has not been shown to be transcribed and translated in vivo, and no such TTV protein has until now been identified, let alone that a function has been assigned to it, at least not a function related to apoptotic activity.
In a preferred embodiment, the invention provides an isolated or recombinant nucleic acid or functional equivalent thereof encoding and/or capable of expression of a TTV-derived proteinaceous substance capable of providing apoptosis. In particular, the invention provides an isolated or recombinant nucleic acid or functional equivalent or functional fragment thereof from which a proteinaceous substance according to the invention can be transcribed and translated.
In another embodiment, the invention provides an isolated or recombinant nucleic acid or functional equivalent or fragment thereof encoding a TTV-derived proteinaceous substance capable of providing apoptosis capable of hybridizing to a nucleic acid molecule encoding a TTV-derived proteinaceous substance capable of providing apoptosis as shown in figure 1, in particular encoding a novel protein capable of providing apoptosis or functional equivalent or functional fragment thereof called TTV-apoptosis protein, abbreviated as TAP.
In particular, the invention provides an isolated or recombinant nucleic acid or functional equivalent or fragment thereof encoding, and preferably capable of expressing a TAP substance capable of providing apoptosis being at least 60% homologous, preferably at least 70%, more preferably at least 80%, even more preferably at least 90%, most preferably at least 95% homologous to a nucleic acid molecule, or to a functional equivalent or functional fragment thereof, encoding a TAP proteinaceous substance as shown in figure 1. Furthermore, the invention provides a vector comprising a nucleic acid encoding a proteinaceous substance according to the invention. Examples of such a vector are given in the detailed description given herein; such as vector pCMV-TAP, a plasmid expressing a TAP product, and so on.
In yet another embodiment, the invention provides a vector comprising a nucleic acid encoding a proteinaceous substance according to the invention, said vector comprising a gene-delivery vehicle, making the invention very useful in gene therapy. By equipping a gene delivery vehicle with a nucleic acid according to the invention, and by targeting said vehicle to a cell or cells that behave over-proliferating and/or have shown decreased death rates, said gene delivery vehicle provides said cell or cells with the necessary means of apoptosis, providing far reaching therapeutic possibilities.
Furthermore, the invention provides a transformed host cell comprising a (recombinant) nucleic acid encoding a proteinaceous substance or a vector according to the invention. Examples comprise transformed or transfected bacterial cells as described in the detailed description herein. Preferred is a host cell according the invention which is a transformed eukaryotic cell, such as mammalian cells transformed or transfected with a nucleic acid or vector according to the invention. Said cells are in general capable to express or to produce a proteinaceous substance capable of providing apoptosis.
The invention furthermore provides an isolated or recombinant TAP proteinaceous substance capable of inducing apoptosis. Expression of such TAP substances in cells, induces the apoptotic process. It can do so alone, or in the presence of other apoptosis inducing substances such as apoptin, and especially so independent of p53 and/or insensitive to anti-apoptosis proteins such as Bcl-2, showing that also in those cases where (functional) p53 is absent and/or active Bcl-2 is present apoptosis can be induced by a substance according to the invention.

In particular, the invention provides a proteinaceous substance according to the invention encoded by a nucleic acid according to the invention, for example comprising at least a part of an amino acid sequence as shown in figure 2 or a functional equivalent or functional fragment thereof capable of providing apoptosis alone or in combination with other apoptosis inducing substances such as apoptin.
The invention also provides an isolated or synthetic antibody specifically recognizing a proteinaceous substance or functional equivalent or functional fragment thereof according to the invention. Such an antibody is for example obtainable by immunizing an experimental animal with a TAP proteinaceous substance or an immunogenic fragment or equivalent thereof and harvesting polyclonal antibodies from said immunized animal, or obtainable by other methods known in the art such as by producing monoclonal antibodies, or (single chain) antibodies or binding proteins expressed from recombinant nucleic acid derived from a nucleic acid library, for example obtainable via phage display techniques.
With such an antibody, the invention also provides a proteinaceous substance specifically recognizable by such an antibody according to the invention, for example obtainable via immunoprecipitation, Western Blotting, or other immunological techniques known in the art.
Furthermore, the invention provides use of a nucleic acid, vector, host cell, or proteinaceous substance according to the invention for the induction of apoptosis. In particular, such use is also provided further comprising use of a nucleic acid encoding apoptin or a functional equivalent or fragment thereof or use of apoptin or a functional equivalent or fragment thereof. Combining these apoptosis-inducing substances increases the percentage of apoptosis in treated tumor cells (see for example figure 4). In particular, such use is provided wherein said apoptosis is p53-independent and/or Bcl-2 insensitive.

Such use as provided by the invention is particularly useful from a therapeutic viewpoint. The invention provides herewith a pharmaceutical composition comprising a nucleic acid, vector, host cell, or proteinaceous substance according to the invention. In addition, such a pharmaceutical composition according to the invention is provided further comprising a nucleic acid encoding apoptin or a functional equivalent or fragment thereof or apoptin or a functional equivalent or fragment thereof.

Such a pharmaceutical composition is in particular provided for the induction of apoptosis, for example wherein said apoptosis is p53-independent and/or Bcl-2 insensitive, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. Also, use of a nucleic acid, vector, host cell, or proteinaceous substance according to the invention is provided for the preparation of a pharmaceutical composition for the treatment of a disease where enhanced cell proliferation or decreased cell death is observed, as is in general the case when said disease comprises cancer or auto-immune disease.
The invention will be explained in more detail in the following detailed description, which is not limiting the invention.

### Detailed description

The invention provides a method for inducing apoptosis through interference with the function of this newly discovered apoptosis-related TAP protein or functional equivalents or fragments thereof and/or the induction of apoptosis by means of (over)expression of TAP or related gene or functional equivalents thereof.

The invention also provides an anti-tumor therapy based on the interference with the function of TAP-like proteins and/or its (over)expression. The invention provides a therapy for cancer, auto-immune diseases or related diseases which is based on TAP-like proteins alone or in combination with apoptin and/or apoptin-like compounds.

### DNA cloning

The various cloning steps have been carried out according to the methods described by Maniatis et al., 1982.

For drug selections Luria Broth (LB) plates for E.coli were supplemented with ampicillin (50 microgram per ml).

### Sequence analysis

The clones containing the sequences encoding TAP proteins were sequenced using dideoxy NTPs according to the Sanger-method, which was performed by BaseClear, Leiden, The Netherlands. The used sequencing primer was a 20-mer of the DNA-sequence 5'-ggatccctagcaggtctgcg-3'.

### Transfection methods

Plasmid DNA was purified by QIAGEN-column chromatography according to the instructions of the manufacturer (Westburg BV, Leusden, The Netherlands).
The various mammalian cell lines were grown in the appropriate culture medium supplemented with penicillin and streptomycin (GIBCO/BRL).
The cells were transfected by calcium-phosphate precipitation as described (Graham and Van der Eb, 1973) or transfected with Fugene, according the instructions provided by the manufacturer (Roche, Almere, The Netherlands).

### Immunofluorescence assays

Cells were fixed with 80% acetone and used for immunofluorescence tests with myc-tagged- and/or apoptin-specific monoclonal antibodies with fluorescein-isothiocyanate (Noteborn et al., 1998).

### Apoptosis assays

The apoptotic cells were initially determined by means of DAPI staining (Telford et al., 1992). DAPI stains intact cellular DNA strongly and regularly, whereas it stains apoptotic DNA weakly and/or irregularly.
The TUNEL assay was carried out as described by Pietersen et al., 1999 with the use of an in-situ cell death detection kit (Roche, Almere, The Netherlands).

### Western-blot analysis

The cells were harvested after transfection and lysed in 2 x sample buffer [100 mM TrisHCl (pH 6.8), 4% SDS, 20% glycerol, 0.2% bromophenol blue, 2.5 % beta-mercaptoethanol]. Proteins were fractionated on 15% SDS-polyacrylamide gels and electroblotted onto Immobilon-P membranes (Millipore). Parts of the blots were then incubated with Mab 9E10 (1:5000) to myc-tag to detect TAP. Positive signals were visualized by enhanced chemiluminescence according to the manufacture's protocol (Amersham, The Netherlands).

### Results and discussion

### Construction of a plasmid containing the TAP gene.

To examine whether TAP protein harbors apoptotic activity we have to make a DNA construct containing the complete TAP DNA sequences, which are shown in Figure 1. In addition, the stop codon has been also indicated. The presented TAP DNA sequence synthesizes a putative protein of 105 amino acids (Figure 2). Furthermore, at the N-terminal end of the TAP gene, a Myc-tag (amino acids: EQKLISEEDL) has been linked which is in frame with the TAP gene product. This Myc-tag enables the recognition of the TAP proteins by means of the Myc-tag-specific 9E10 antibody.

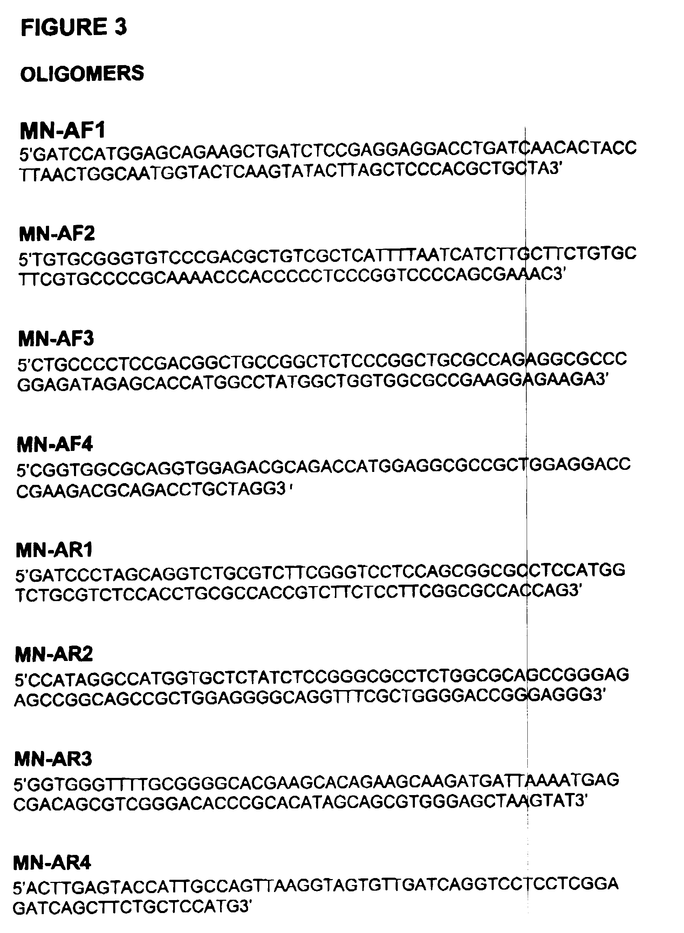

The DNA construct containing the TAP DNA sequences, Myc-tag DNA sequences and BamH1 restriction-enzyme sites has been made by using a panel of DNA oligomers as shown in Figure 3. All steps for making the required approximately 0.36 kb DNA fragment was carried by The Midland Certified Reagent Co., Midland, Texas, USA.
In principle the synthesized DNA oligomers were synthesized and purified by preparative polyacrylamide gelelectrophoresis and 5'-phosphorylated to permit ligation. Subsequently, the various oligomers were annealed and then ligated en masse in a thermostable ligation reaction. The long internal overlaps annealed stably and are efficiently ligated, whereas the cohesive termini tend to remain dissiciated. As a consequence, the ligation product could be directly purified by preparative low-melting-point agarose gel electrophoresis using the freeze-squeeze method. The appropriated approximately 0.36 DNA fragment was made visible in the gel with long wavelength UV light. Next, the DNA band was excised and purified and ligated in BamH1-linearized pUc19 plasmid DNA, which was treated with calf intestine alkaline phosphatase. The final product was analyzed by BamH1 digestion for containing an approximately 0.36 DNA fragment and sequence analysis. The plasmid DNA containing the myc-tagged TAP DNA sequences has been called pMN-TAP.

Construction of an expression vector for the identification of TAP protein in human and other mammalian cells.
To study whether the cloned myc-tagged TAP gene encode protein products, we have carried out the following experiments.
The DNA plasmid pcDNA3.1 (In Vitrogen, Groningen, The Netherlands) contains the cytomegalovirus (CMV) promoter enabling high levels of expression of foreign genes in transformed human and other mammalian cells, such as Saos-2 and Cos cells.

The pcDNA3.1 construct expressing Myc-tagged TAP DNA was constructed as follows. The pMN-TAP clone was digested with the restriction enzyme BamH1 and the requested approximately 0.36 kb DNA insert was isolated. The expression vector pcDNA3.1 digested with BamH1 and treated with calf intestine alkaline phosphatase and ligated to the isolated myc-tagged TAP DNA insert. By restriction-enzyme digestions and sequence analysis, the pcDNA3.1 construct containing the myc-tagged TAP DNA in the correct orientation was identified. The final construct containing the TAP sequences is called pCMV-TAP.

The synthesis of the Myc-tagged TAP protein was analyzed by transfection of Cos cells with plasmid pCMV-TAP. As negative control, Cos cells were mock-transfected. Two days after transfection, the cells were lysed and Western-blot analysis was carried out using the Myc-tag-specific antibody 9E10. The Cos cells transfected with pCMV-TAP were proven to synthesize a specific Myc-tagged TAP product with the expected size of approximately 12 kDa. As expected, the lysates of the mock-transfected Cos cells did not contain a protein product reacting with the Myc-tag-specific antibodies.

These results indicate that we have been able to construct a plasmid that is able to produce a protein product derived from the putative TAP open reading frame.

### Over-expression of the novel TAP protein in human hepatoma HepG2 cells induces the apoptotic process.

Next, we have examined whether TAP carries apoptotic activity. First, we have analyzed the cellular localization of the novel TAP protein in human hepatoma-derived HepG2 cells, which are synthesizing functional tumor suppressor protein p53 (Pietersen et al., 1999). To that end, the HepG2 cells were transfected with plasmid pCMV-TAP encoding the myc-tagged TAP protein, as described by Danen-van Oorschot (1997). As a negative control, HepG2 cells were transfected with a plasmid encoding myc-tagged lacZ.
Two days after transfection, the cells were fixed. By indirect immunofluorescence using the myc-tag-specific antibodies 9E10 and DAPI, which stains the nuclear DNA, it was shown that the TAP protein was present in the nucleus of the cell. Actually, it co-localizes with the chromatin/DNA structures.
Finally, we examined whether (over)-expression of our novel TAP protein results in induction of apoptosis. To that end, we have analyzed the apoptin-positive cells for DAPI-staining. Four days after transfection, almost all TAP-positive cells were aberrantly stained with DAPI, which is indicative for induction of apoptosis (Telford, 1992, Danen-van Oorschot, 1997). The nuclear DNA of the cells expressing lacZ was not aberrantly stained with DAPI. Therefore, the obtained results (Figure 4) indicate that TAP can induce apoptosis in human tumor cells.

### Over-expression of the novel TAP protein in human osteosarcoma Saos-2 cells induces the apoptotic process.

During tumor development, most of the tumors will lack functional tumor suppressor p53. Tumor cells lacking functional p53 are in general poor responders to (chemo)therapeutic agents. In the above described experiment, we have shown that TAP can induce apoptosis in cells containing functional tumor suppressor protein p53. Therefore, it is also of importance to prove whether TAP can induce apoptosis in human tumor cells in the absence of functional p53. To that end, we have transfected human osteosarcoma-derived Saos-2 cells synthesizing no tumor suppressor p53 protein (Zhuang et al., 1995) with plasmid pCMV-TAP encoding myc-tagged TAP protein. As control, Saos-2 cells were transfected with the plasmid pCMV-lacZ. Four days after transfection, the cells were fixed and analyzed for myc-tagged TAP expression by means of immunofluorescence using the myc-tag-specific antibody 9E10 and apoptotic activity by DAPI staining. In parallel, transfected Saos-2 cells were fixed and examined for induction of apoptosis by means of a TUNEL assay.
Most of the TAP-positive Saos-2 cells were stained aberrantly with DAPI whereas cells containing lacZ were not, which is indicative for induction of TAP-specific apoptosis. In addition, cell cultures transfected with pCMV-TAP showed a significant higher amount of TUNEL-positive cells than Saos-2 cell cultures transfected with pCMV-lacZ.

The results (Figure 4) obtained with TAP synthesis in both HepG2 and Saos-2 cells prove that TAP can induce apoptosis independent of p53. Therefore, tumors or tumorous cells having functional p53 or not having functional p53 can be treated with TAP-synthesizing vectors.

### Over-expression of the novel TAP protein in human lymphoma DoHH-2 cells induces the apoptotic process.

Besides non-functional p53, over expression of anti-apoptosis proteins such as Bcl-2 hamper conventional anti-cancer therapies. Therefore, we have also examined whether TAP can induce apoptosis in human tumor cells over-expressing Bcl-2 such as DoHH-2 cells (Zhuang et al., 1995). To that end, DoHH-2 cells were transfected with pCMV-TAP or pCMV-lacZ and analyzed as described for the HepG2 cells. Only the DoHH-2 cells synthesizing TAP protein were shown to be stained aberrantly with DAPI. Therefore, we conclude that TAP also induces apoptosis in cells over-expressing Bcl-2. The results are shown in Figure 4.

### Co-expression of TAP and apoptin induces apoptosis in human tumor cells in a very efficient way.

The CAV-derived protein apoptin induces apoptosis upon nuclear localization as seems also to be the case for TAP-induced apoptosis. In the following transfection experiments (carried out as described above), we have examined the rate of apoptosis induction in human tumor cells synthesizing both TAP and apoptin in comparison with tumor cells synthesizing only TAP or apoptin.

All 3 examined human tumor cell lines HepG2, Saos-2 and DoHH-2 underwent a significant higher level of apoptosis after co-expression of apoptin and TAP proteins in comparison with the cells expressing apoptin or TAP separately. The results are shown in Figure 4.

In conclusion, we have provided evidence that expression of TAP proteins in various human tumorigenic cells results in induction of apoptosis. Therapies based on induction of (p53-independent and/or Bcl-2 insensitive) apoptosis are possible utilizing the TAP proteins. Combined therapies can be carried out using TAP protein and other apoptosis-inducing proteins such as apoptin. Another CAV-derived protein, which is known to induce apoptosis and also known to enhance apoptin activity is VP2 (Noteborn et al., 1997).

### Functional domains of TAP

Although, TAP does not share overall sequence similarities with any known protein sequence and in particular with the CAV-derived apoptosis-inducing protein apoptin, it also has apoptotic activity in human tumor cells. TAP harbors putative sequences resembling a nuclear location signal or a nuclear export signal as indicated in Figure 2. Furthermore, TAP has a relative high percentage (>8%) of proline residues.
Computer analysis revealed a significant structural homology among TAP and apoptin, which might explain the similarity in their activity.

### Description of the figures

Figure 1 shows the DNA sequence encoding TAP protein which has apoptotic activity in (human) tumor cells.

Figure 2 shows an amino-acid sequence of TAP protein. The NES domain is located on the TAP protein in between amino acids 27 and 39. The NLS domain is situated in the TAP protein from position 62 and 68.

Figure 3 shows the scheme of the used oligomers to construct pMN-TAP. Besides the TAP sequence the oligomers contain a myc-tag and BamH1 restriction-enzyme site, which are required for binding to the myc-tag-specific antibody 9E10 and enabling cloning in a BamH1 restriction-enzyme site, respectively.

Figure 4 shows the apoptotic activity of TAP protein and/or apoptin in various human tumor cell lines. (-): no apoptotic activity; (+): strong apoptotic activity; (++ and +++): very strong apoptotic activity.
DoHH-2: human lymphoma-derived cell line over-expressing Bcl-2; HepG2: human hepatoma-derived cell line expressing wild-type p53; Saos-2: human osteosarcoma-derived cells lacking functional p53.

### References

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Charlton, M., Adjei, P., Poterucha, J., Zein, N., Moore, B., Therneau T., Krom, R., and Wiesner, R. 1998. TT-virus infection in North American blood donors, patients with fulminant hepatic failure, and cryptogenic cirrhosis. Hepatology 28, 839-842.
3. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
4. Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.
5. Duke, R.C., Ocjius, D.M., Young, J, D-E. (1996). Cell suicide in health and disease. Scientific American December 1996, 48-55.
6. Earnshaw, W.C., 1995. Nuclear changes in apoptosis. Current Opinion in Cell Biology 7, 337-343.
7. Hockenberry, D.M. (1994). Bcl-2 in cancer, development and apoptosis. Journal of Cell Science, Supplement 18, 51-55.
8. Kerr, J.F.R., Winterford, C.M., and Harmon, B.V. (1994). Apoptosis: Its significance in cancer and cancer therapy. Cancer 73, 2013-2026.
9. Levine, A.J. (1997). p53, the cellular gatekeeper for growth and division. Cell 88, 323-331.
10. Maniatis, T., Fritsch, E.F., and Sambrook, J. (1982).Molecular Cloning: A Laboratory Manual. CSHL Press, New York, USA.
11. McDonell T.J., Meyn, R.E., Robertson, L.E. (1995). Implications of apoptotic cell death regulation in cancer therapy. Seminars in Cancer Biology 6, 53-60.
12. Miyata, H., Tsunoda, H., Kazi, A. Yamada, A., Khan, M.A., Murakami, J., Kamahora, T., Shiraki, K., and Hino, S. (1999). Indentification of a novel GC-rich 113-nucleotide region to complete the circular, single-stranded DNA genome of TT virus, the first human circovirus. Journal of Virology 73, 3582-3586.
13. Noteborn, M.H.M. (1996). PCT application WO 96/41191. Apoptin induces apoptosis in human transformed and malignant cells but not in normal cells as essential characteristic for the development of an anti-tumor therapy.
14. Noteborn, M.H.M., and.De Boer, G.F. (1996). Patent USA/no. 030, 335.
15. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
16. Noteborn, M.H.M., and Pietersen, A. (1998). A gene delivery vehicle expressing the apoptosis-inducing proteins VP2 and/or apoptin. PCT Application no. NL98/00213.
17. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
18. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology. 79, 3073-3077.
19. Noteborn, M.H.M., and Zhang, Y. (1998). Methods and means for determining the transforming capability of agents, for determining the predisposition of cells to become transformed and prophylactic treatment of cancer using apoptin-like activity. PCT Application NL98/00457.
20. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
21. Paulovich, A.G., Toczyski, D., Hartwell, H. (1997). When checkpoints fail. Cell 88, 315-321.
22. Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the apoptin gene. Gene Therapy 6, 882-892.
23. Rose, M.D., Winston, F., and Hieter, P. (1990). Methods in yeast genetics. A laboratory course manual. Cold Spring Harbor Laboratory Press, Cold Spring Harbor, New York, USA.
24. Sachs, L. and Lotem, J. (1993). Control of programmed cell death in normal and leukemia cells: New implications for therapy. Blood 82, 15-21.
25. Sanger, F., Nicklen, S., and Coulsen, A.R. (1977). DNA sequencing with chain-terminating inhibitors. Proceedings National Academic Sciences USA 74, 5463-5467.
26. Steller, H. (1995). Mechanisms and genes of cellular suicide. Science 267, 1445-1449.
27. Telford, W.G., King, L.E., Fraker, P.J. (1992). Comparative evaluation of several DNA binding dyes in the detection of apoptosis-associated chromatin degradation by flow cytometry. Cytometry 13, 137-143.
28. Teodoro, J.G. and Branton, P.E. (1997). Regulation of apoptosis by viral gene products. Journal of Virology 71, 1739-1746.
29. Thompson, C.B. (1995). Apoptosis in the pathogenesis and treatment of disease. Science 267, 1456-1462.
30. White, E. (1996). Life, death, and the pursuit of apoptosis. Genes and development 10, 1-15.
31. Wyllie, A.H. (1995). The genetic regulation of apoptosis. Current Opinion in Genetics and Development 5, 97-104.
32. Wyllie, A.H., Kerr, J.F.R., Currie, A.R. (1980). Cell death: The significance of apoptosis. International Review of Cytology 68, 251-306.
33. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.
34. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. Apoptosis inducing proteinaceous substance encoded by an isolated or recombinant nucleic acid or functional equivalent or fragment thereof derivable from mammalian gyrovirus-like virus nucleic acid.

2. Proteinaceous substance according to claim 1 wherein said mammalian gyrovirus-like virus comprises a single-stranded circular DNA virus.

3. Proteinaceous substance according to claim 2 wherein said single-stranded circular DNA virus comprises a TTV-like virus.

4. Proteinaceous substance according to anyone of claims 1 to 3 wherein said isolated or recombinant nucleic acid is at least 60 per cent homologous to a TTV nucleic acid as shown in figure 1

5. Proteinaceous substance according to anyone of claims 1 to 4 capable of providing apoptosis further in combination with another apoptosis-inducing substance.

6. Proteinaceous substance according to claim 5 wherein said apoptosis-inducing substance comprises apoptin or a functional fragment thereof.

7. Proteinaceous substance according to anyone of claims 1 to 6 capable of providing apoptosis independent of p53.

8. Proteinaceous substance according to anyone of claims 1 to 7 comprising a nuclear location signal and/or a nuclear export signal.

9. An isolated or recombinant nucleic acid or functional equivalent or functional fragment thereof from which a proteinaceous substance according to anyone of claims 1 to 8 can be transcribed and translated.

10. A vector comprising a nucleic acid according to claim 9.

11. A vector according to claim 10 comprising a gene-delivery vehicle.

12. A host cell comprising a nucleic acid according to claim 10 or a vector according to claim 11.

13. A host cell according to claim 12 which is a eukaryotic cell such as a yeast cell or a vertebrate cell.

14. An isolated or synthetic antibody specifically recognising a proteinaceous substance or functional equivalent or functional fragment thereof according to anyone of claims 1 to 8.

15. A proteinaceous substance specifically recognisable by an antibody according to claim 14.

16. Use of a nucleic acid according to claim 9, a vector according to claims 10 or 11, a host cell according to claim 13 or 14, a proteinaceous substance according to anyone of claims 1 to 8 or 15 for the induction of apoptosis.

17. Use according to claim 16 wherein said apoptosis is p53-independent.

18. Use according to claim 16 or 17 further comprising use of a nucleic acid encoding apoptin or a functional equivalent or fragment thereof or use of apoptin or a functional equivalent or fragment thereof.

19. A pharmaceutical composition comprising a nucleic acid according to claim 9, a vector according to claims 10 or 11, a host cell according to claim 13 or 14, a proteinaceous substance according to anyone of claims 1 to 8 or 15

20. A pharmaceutical composition according to claim 19 further comprising a nucleic acid encoding apoptin or a functional equivalent or fragment thereof.

21. A pharmaceutical composition according to claim 19 or 20 for the induction of apoptosis.

22. A pharmaceutical composition according to claim 21 wherein said apoptosis is p53-independent.

23. A pharmaceutical composition according to anyone of claims 19 to 22 for the treatment of a disease where enhanced cell proliferation or decreased cell death is observed.

24. A pharmaceutical composition according to claim 23 wherein said disease comprises cancer or auto-immune disease.

25. A method for treating an individual carrying a disease where enhanced cell proliferation or decreased cell death is observed comprising treating said individual with a pharmaceutical composition according to anyone of claims 19 to 24.

26. Use of a nucleic acid according to claim 9, a vector according to claims 10 or 11, a host cell according to claim 13 or 14, a proteinaceous substance according to anyone of claims 1 to 8 or 15 for the preparation of a pharmaceutical composition for the treatment of a disease where enhanced cell proliferation or decreased cell death is observed.


### Annex to the application documents- subsequently filed sequences listing

SEQUENCE LISTING

<110> Leadd B.V.

<120> Apoptosis inducing proteinaceous substance

<130> P51373EP00

<140> 99203467.8
<141> 1999-10-21

<160> 11

<170> PatentIn Ver. 2.1

<210> 1
<211> 20
<212> DNA
<213> Artificial Sequence

<220>
<223> Description of Artificial Sequence: sequencing primer

<400> 1
ggatccctag caggtctgcg                                              20

<210> 2
<211> 318
<212> DNA
<213> TTV-like virus

<220>
<223> Description of Combined DNA/RNA Molecule: TAP protein of TTV-like virus

<220>
<221> CDS
<222> (1)..(318)
<223> /note="TAP-protein of TTV-like virus"

<400> 2
atg atc aac act acc tta act ggc aat ggt act caa gta tac tta gct    48
Met Ile Asn Thr Thr Leu Thr Gly Asn Gly Thr Gln Val Tyr Leu Ala
  1               5                  10                  15

ccc acg ctg cta tgt gcg ggt gtc ccg acg ctg tcg ctc att tta atc    96
Pro Thr Leu Leu Cys Ala Gly Val Pro Thr Leu Ser Leu Ile Leu Ile
             20                  25                  30

atc ttg ctt ctg tgc ttc gtg ccc cgc aaa acc cac ccc ctc ccg gtc    144
Ile Leu Leu Leu Cys Phe Val Pro Arg Lys Thr His Pro Leu Pro Val
         35                  40                  45

ccc agc gaa acc tgc ccc tcc gac ggc tgc cgg ctc tcc cgg ctg cgc    192
Pro Ser Glu Thr Cys Pro Ser Asp Gly Cys Arg Leu Ser Arg Leu Arg
     50                  55                  60

cag agg cgc ccg gag ata gag cac cat ggc cta tgg ctg gtg gcg ccg    240
Gln Arg Arg Pro Glu Ile Glu His His Gly Leu Trp Leu Val Ala Pro
 65                  70                  75                  80

aag gag aag acg gtg gcg cag gtg gag acg cag acc atg gag gcg ccg 288
Lys Glu Lys Thr Val Ala Gln Val Glu Thr Gln Thr Met Glu Ala Pro
85 . 90 95

ctg gag gac ccg aag acg cag acc tgc tag 318
Leu Glu Asp Pro Lys Thr Gln Thr Cys
100 105

<210> 3
<211> 105
<212> PRT
<213> TTV-like virus
<223> Description of Combined DNA/RNA Molecule: TAP protein of TTV-like virus

<400> 3
Met Ile Asn Thr Thr Leu Thr Gly Asn Gly Thr Gln Val Tyr Leu Ala
1               5                   10                  15

Pro Thr Leu Leu Cys Ala Gly Val Pro Thr Leu Ser Leu Ile Leu Ile
            20                  25                  30

Ile Leu Leu Leu Cys Phe Val Pro Arg Lys Thr His Pro Leu Pro Val
        35                  40                  45

Pro Ser Glu Thr Cys Pro Ser Asp Gly Cys Arg Leu Ser Arg Leu Arg
    50                  55                  60

Gln Arg Arg Pro Glu Ile Glu His His Gly Leu Trp Leu Val Ala Pro
65                  70                  75                  80

Lys Glu Lys Thr Val Ala Gln Val Glu Thr Gln Thr Met Glu Ala Pro
                85                  90                  95

Leu Glu Asp Pro Lys Thr Gln Thr Cys
            100                 105

<210> 4
<211> 95
<212> DNA
<213> Artificial Sequence

<220>
<223> Description of Artificial Sequence: MN-AF1 oligomers to construct pMN-TAP

<400> 4
gatccatgga gcagaagctg atctccgagg aggacctgat caacactacc ttaactggca 60
atggtactca agtatactta gctcccacgc tgcta 95

<210> 5
<211> 95
<212> DNA
<213> Artificial Sequence

<220>
<223> Description of Artificial Sequence: MN-AF2 oligomers to construct pMN-TAP

<400> 5

tgtgcgggtg tcccgacgct gtcgctcatt ttaatcatct tgcttctgtg cttcgtgccc 60
cgcaaaaccc accccctccc ggtccccagc gaaac 95

<210> 6
<211> 95
<212> DNA
<213> Artificial Sequence

<220>
<223> Description of Artificial Sequence: MN-AF3 oligomers to contruct pMN-TAP

<400> 6
ctgcccctcc gacggctgcc ggctctcccg gctgcgccag aggcgcccgg agatagagca 60
ccatggccta tggctggtgg cgccgaagga gaaga 95

<210> 7
<211> 69
<212> DNA
<213> Artificial Sequence

<220>
<223> Description of Artificial Sequence: MN-AF4 oligomers to construct pMN-TA[?]

<400> 7
cggtggcgca ggtggagacg cagaccatgg aggcgccgct ggaggacccg aagacgcaga 60
cctgctagg 69

<210> 8
<211> 95
<212> DNA
<213> Artificial Sequence

<220>
<223> Description of Artificial Sequence: MN-AR1 oligomers to construct pMN-TA[?]

<400> 8
gatccctagc aggtctgcgt cttcgggtcc tccagcggcg cctccatggt ctgcgtctcc 60
acctgcgcca ccgtcttctc cttcggcgcc accag 95

<210> 9
<211> 95
<212> DNA
<213> Artificial Sequence

<220>
<223> Description of Artificial Sequence: MN-AR2 oligomers to construct pMN-TA[?]

<400> 9
ccataggcca tggtgctcta tctccgggcg cctctggcgc agccgggaga gccggcagcc 60
gctggagggg caggtttcgc tggggaccgg gaggg 95

<210> 10
<211> 95
<212> DNA
<213> Artificial Sequence

<220>
<223> Description of Artificial Sequence: MN-AR3 oligomers to construct pMN-TAP

<400> 10
ggtgggtttt gcggggcacg aagcacagaa gcaagatgat taaaatgagc gacagcgtcg 60
ggacacccgc acatagcagc gtgggagcta agtat 95

<210> 11
<211> 69
<212> DNA
<213> Artificial Sequence

<220>
<223> Description of Artificial Sequence: MN-AR4 oligomers to construct pMN-TAP

<400> 11
acttgagtac cattgccagt taaggtagtg ttgatcaggt cctcctcgga gatcagcttc 60
tgctccatg 69